# FASCICULE DE BREVET EUROPEEN

(11) **EP 2 345 401 B1**
(45) Date de publication et mention de la délivrance du brevet: **22.07.2015**
(21) Numéro de dépôt: 10196185.2
(22) Date de dépôt: 21.12.2010
(51) Int. Cl.: A61K 8/31, A61K 8/37, A61K 8/891, A61Q 5/12

(54) **Utilisation pour le traitement cosmétique des fibres kératiniques d'alcane(s) linéaire(s) volatil(s) et d'ester(s) gras liquide(s) en milieu anhydre, et composition cosmétique anhydre à base d'alcane(s) linéaire(s) volatil(s), d'ester gras liquide(s) et d'huile(s) particulière(s)**
Verwendung von flüchtigem/en linearem/en Alkan/en und flüssigem/en Fettester/n in einem wasserfreien Milieu zur kosmetischen Behandlung von Keratinfasern, und wasserfreie kosmetische Zusammensetzung auf der Basis von flüchtigem/en linearem/en Alkan/en, flüssigem/en Fettester/n und speziellem/en Öl/en.
Use of volatile linear alkanes and liquid fatty esters in an anhydrous medium for cosmetic treatment of keratin fibres, and anhydrous cosmetic composition of volatile linear alkanes, liquid fatty esters and specific oils.

(30) Priorité: 23.12.2009 FR 0959483
(43) Date de publication de la demande: 20.07.2011
(73) Titulaire: L'Oréal, 75008 Paris (FR)
(72) Inventeur: Desenne, Patricia, 74370, Pringy (FR); Bourdin, Claire, 92300, Levallois Perret (FR); Gringore, Charles, 92600, Asnieres Sur Seine (FR)
(74) Mandataire: Dossmann, Gérard

(56) Documents cités:
- DE-A1-102008 012 457

## Description

La présente invention concerne l'utilisation pour le traitement cosmétique des fibres kératiniques, comme les cheveux, d'un ou plusieurs alcane(s) linéaire(s) volatil(s) et d'un ou plusieurs ester(s) gras liquide(s) en un rapport pondéral alcane(s) linéaire(s) volatil(s)/ester(s) gras liquide(s) inférieur à 5, en milieu anhydre, une composition cosmétique anhydre à base d'alcane(s) linéaire(s) volatil(s), d'ester(s) gras liquide(s) et d'huile(s) particulière(s), le rapport pondéral alcane(s) linéaire(s) volatil(s)/ester(s) gras liquide(s) étant inférieur à 5, et un procédé de traitement cosmétique mettant en oeuvre ladite composition.

Dans le domaine du traitement capillaire, l'utilisation de solvants volatils est connue dans des produits capillaires de soins et de brillance. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs.

Ces solvants volatils qui sont généralement des esters gras liquides, des huiles hydrocarbonées de type isododécane ou isohexadécane, et/ou des huiles siliconées, peuvent notamment conduire à des problèmes de toucher gras, de manque de brillance, et de cheveux raidis et durs

En outre, ces solvants volatils sont généralement présents en forte concentration dans les produits anhydres. Ils peuvent présenter alors l'inconvénient d'avoir un impact négatif sur l'environnement, notamment sur le milieu aquatique.

Il subsiste donc un besoin de remplacer ces solvants volatils pour éviter les inconvénients cités ci-dessus.

La demanderesse a maintenant découvert de façon inattendue et surprenante, que l'utilisation d'un ou plusieurs alcane(s) linéaire(s) volatil(s) et d'un ou plusieurs ester(s) gras liquide(s) de mono- ou diacide carboxylique en C₁₋₃₀ et de monoalcool en C₁₋₃₀ en milieu anhydre, dans un rapport pondéral alcane(s) linéaire(s) volatil(s)/ester(s) gras liquide(s) inférieur à 5, permettait d'éviter un transfert du produit sur les mains après passage des mains dans les cheveux et les inconvénients mentionnés ci-dessus, et d'améliorer les propriétés cosmétiques des cheveux telles que le lissage, la brillance, une facilité de mise en forme, le démêlage, la légèreté, la souplesse et le toucher.

En particulier, l'application sur cheveux humides permet d'obtenir des cheveux plus lisses et plus brillants. En outre, lorsque la composition est utilisée sur cheveux secs, on obtient des cheveux plus brillants, que l'on peut mettre plus facilement en forme.

Ainsi, l'invention a pour objet l'utilisation pour le traitement cosmétique des fibres kératiniques, comme les cheveux, d'un ou plusieurs alcanes linéaires volatils comportant de 7 à 15 atomes de carbone et d'un ou plusieurs esters gras liquides à 25°C et pression atmosphérique de mono- ou diacide carboxylique en C₁₂₋₃₀ et de monoalcool en C₁₋₃₀, en milieu anhydre, dans un rapport pondéral alcane(s) linéaire(s) volatil(s)/ester(s) gras liquide(s) inférieur à 5.

Elle a encore pour objet une composition cosmétique comprenant, dans un milieu anhydre :
un ou plusieurs alcanes linéaires volatils comportant de 7 à 15 atomes de carbone,
- un ou plusieurs esters gras liquides à 25°C et pression atmosphérique d'un mono- ou diacide carboxylique en C₁₂₋₃₀ et d'un mono-alcool en C₁₋₃₀, en un rapport pondéral alcane(s) linéaire(s) volatil(s)/ester(s) gras liquide(s) supérieur ou égal à 0,1 et inférieur à 5, et
- une ou plusieurs huiles choisies parmi les huiles siliconées.

Un autre objet de l'invention est un procédé de traitement cosmétique des matières kératiniques, telles que les fibres kératiniques, par exemple les cheveux, mettant en oeuvre ladite composition.

Selon l'invention, un ou plusieurs alcanes linéaires volatils comportant de 7 à 15 atomes de carbone sont utilisés en combinaison avec un ou plusieurs esters gras liquides de mono- ou diacide carboxylique en C₁₂₋₃₀ et de monoalcool en C₁₋₃₀, en milieu anhydre, dans un rapport pondéral alcane(s) linéaire(s) volatil(s)/ester(s) gras liquide(s) inférieur à 5, pour le traitement cosmétique des fibres kératiniques, comme les cheveux. Cette utilisation conduit notamment à l'obtention de meilleures propriétés de lissage, de brillance, de facilité de mise en forme, de démêlage, de légèreté, de souplesse et/ou de toucher.

Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire, susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, les alcanes linéaires volatils convenant à l'invention ont une pression de vapeur allant de 0,3 à 2000 Pa, mieux de 0,3 à 1000 Pa, à température ambiante (25°C).

De façon plus préférée, les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, les alcanes linéaires volatils convenant dans l'invention peuvent être les alcanes linéaires comportant de 8 à 14 atomes de carbone et mieux de 9 à 14 atomes de carbone.

De façon plus préférée, les alcanes linéaires volatils convenant dans l'invention peuvent être les alcanes linéaires comportant de 10 à 14 atomes de carbone, et encore plus préférentiellement de 11 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14). En particulier, l'isotope ¹⁴C peut être présent en un rapport en nombre d'isotopes ¹⁴C/¹²C (ou ratio ¹⁴C/¹²C) supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C/ ¹²C va de 6.10⁻¹³ à 1,2.10⁻¹².

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets WO 2007/068371 et WO2008/155059. Ces alcanes sont obtenus à partir d'alcools gras, eux-mêmes obtenus à partir d'huile de coprah ou de palme.

Les alcanes linéaires volatils convenant à l'invention sont le n- heptane (C7), le n-octane (C8), le n-nonane (C9), le n-décane (C10), le n-undécane (C11), le n-dodécane (C12), le n-tridécane (C13), le n-tétradécane (C14), le n-pentadécane (C15), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode de réalisation préféré, on peut citer les mélanges de n-undécane (C11) et de n-tridécane (C13) obtenus aux exemples 1 et 2 de la demande WO2008/155059.

On peut également citer le n-dodécane (C12) et le n-tétradécane (C 14) vendus respectivement sous les références PARAFOL 12-97 et PARAFOL 14-97 par la société Sasol, ainsi que leurs mélanges.

Un mode de réalisation consiste à utiliser un seul alcane linéaire volatil.

Alternativement, on peut utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que, par exemple, un mélange d'au moins 3 alcanes linéaires volatils comportant de 7 à 15 atomes de carbone distincts et différant entre eux d'un nombre de carbone d'au moins 1, peuvent être utilisés dans l'invention.

Dans le cas des mélanges de deux alcanes linéaires volatils, lesdits deux alcanes linéaires volatils représentent de préférence plus de 95% et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cₙ avec n allant de 7 à 15
- de 10 à 50% en poids, de préférence de 20 à 45% en poids, de préférence de 24 à 40% en poids d'alcane linéaire volatil en Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés,
lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane) et
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier, un tel mélange peut être obtenu selon l'exemple 1 ou l'exemple 2 de la demande WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

Par « ester gras liquide », on entend au sens de la présente invention, un ester d'acide gras et/ou d'alcool gras liquide à température ambiante (25°C) et à pression atmosphérique (101 325 Pa).

Le ou les ester(s) gras liquide(s) utilisé(s) dans l'invention sont des esters gras d'un mono- ou diacide carboxylique en C₁₂₋₃₀, de préférence en C₁₂₋₂₂, et d'un monoalcool en C₁₋₃₀, de préférence en C₁₋₂₀.

Dans un mode de réalisation de l'invention, l'ester gras liquide comporte de 10 à 50 atomes de carbone au total.

Comme exemples d'esters gras liquides pouvant être utilisés dans la présente invention, on peut notamment citer le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate de 2-octyldodécyle, le monococoate de 2-éthylhexyle (ou monococoate d'octyle), le palmitate d'éthyle, le palmitate d'isopropyle, le palmitate d'isobutyle, le palmitate de 2-éthylhexyle (ou palmitate d'octyle), le stéarate de butyle, le stéarate d'isopropyle, le stéarate d'isobutyle, le stéarate d'isocétyle, l'isostéarate d'isotéaryle, l'isostéarate d'isopropyle, le stéarate de 2-éthylhexyle (ou stéarate d'octyle), l'hydroxystéarate de 2-éthylhexyle (ou hydroxystéarate d'octyle), l'oléate de décyle, le sébacate de diisopropyle, l'isononanoate d'isononyle, le néopentanoate de tridécyle, le néopentanoate d'isocétyle, le néopentanoate d'isostéaryle, le néopentanoate d'octyldodécyle et le néopentanoate d'isoarachidyle et leurs mélanges.

De préférence, l'ester gras liquide utilisé dans l'invention est choisi parmi le myristate d'isopropyle, le myristate de méthyle, le myristate d'éthyle, le myristate de butyle, le myristate d'isobutyle, le myristate de 2-octyldodécyle, le monococoate de 2-éthylhexyle (ou monococoate d'octyle), le palmitate d'éthyle, le palmitate d'isopropyle, le palmitate d'isobutyle, le palmitate de 2-éthylhexyle (ou palmitate d'octyle), le stéarate de butyle, le stéarate d'isopropyle, le stéarate d'isobutyle, le stéarate d'isocétyle, l'isostéarate d'isotéaryle, l'isostéarate d'isopropyle, le stéarate de 2-éthylhexyle (ou stéarate d'octyle), l'hydroxystéarate de 2-éthylhexyle (ou hydroxystéarate d'octyle), l'oléate de décyle et plus particulièrement le myristate d'isopropyle, le palmitate d'isopropyle, et leurs mélanges.

Le rapport pondéral alcane(s) linéaire(s) volatil(s)/ester(s) gras liquide(s) est inférieur à 5, et de préférence supérieur ou égal à 0,1. En particulier, ce dernier va de 0,1 à 4,9, mieux de 0,1 à 4,7.

Par milieu anhydre, on entend au sens de la présente invention un milieu présentant une teneur en eau inférieure à 5% en poids, de préférence inférieure à 2% en poids, de manière encore plus particulière inférieure à 1% en poids, par rapport au poids total de la composition . Il est à noter que l'eau peut aussi se trouver sous forme d'eau liée, comme l'eau de cristallisation des sels ou des traces d'eau adsorbée par les matières premières utilisées dans le cadre de l'invention. Ce milieu peut comprendre un ou plusieurs solvants organiques.

A titre de solvants organiques susceptibles d'être présents dans le milieu anhydre, on peut par exemple citer les alcools, linéaires ou ramifiés, en C₂₋C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol ; ainsi que les alcools aromatiques comme l'alcool benzylique ou le phénoxyéthanol ; et leurs mélanges.

L'invention concerne aussi une composition cosmétique comprenant, dans un milieu anhydre :
- un ou plusieurs alcanes linéaires volatils comportant de 7 à 15 atomes de carbone,
- un ou plusieurs esters gras liquides à 25°C et pression atmosphérique d'un mono- ou diacide carboxylique en C₁₂₋₃₀ et d'un mono-alcool en C₁₋₃₀ en un rapport pondéral alcane(s) linéaire(s) volatil(s)/ester(s) gras liquide(s) supérieur ou égal à 0,1 et inférieur à 5, et
- une ou plusieurs huiles choisies parmi les huiles siliconées.

Le milieu anhydre, les alcanes linéaires volatils, les esters gras liquides et le rapport pondéral alcane(s) linéaire(s) volatil(s)/ester(s) gras liquide(s) sont tels que définis ci-dessus.

La composition de l'invention peut comprendre de 0,5 % à 90 % en poids d'alcane(s) linéaire(s) volatil(s) comportant de 7 à 15 atomes de carbone, en particulier de 1 % à 70 % en poids, et plus particulièrement de 2 % à 70 % en poids, et mieux de 15% à 70% en poids d'alcane(s) linéaire(s) volatil(s) par rapport au poids total de la composition.

L'ester ou les esters gras liquide(s) à 25°C et pression atmosphérique d'un mono- ou diacide carboxylique en C₁₂₋₃₀, mieux en C₁₂₋₂₂, et d'un mono-alcool en C₁₋₃₀, de préférence en C₁₋₂₀ est ou sont présent(s), de préférence en une quantité de 0,1 à 90 % en poids, plus particulièrement de 1 à 80 %, et mieux de 10 à 80 % en poids par rapport au poids total de la composition.

On entend par "huile" tout composé lipophile, non ionique, insoluble dans l'eau et liquide à température ambiante (25 °C) et sous pression atmosphérique (760 mm de Hg, soit 101 325 Pa). Par insoluble dans l'eau, on entend au sens de la présente invention un composé dont la solubilité à pH spontané dans l'eau à 25 °C et à pression atmosphérique est inférieure à 1 % et de préférence inférieure à 0,5 % en poids. Les huiles sont solubles dans des solvants organiques dans les mêmes conditions de température et de pression, comme par exemple le chloroforme, l'éthanol ou le benzène. De plus les huiles sont liquides à la température ordinaire (25°C) et à pression atmosphérique. Les huiles ont de préférence une température de fusion inférieure à 5°C.

Les huiles utilisées dans la présente invention présentent de préférence une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies parmi les huiles siliconées et leurs mélanges.

A titre d'exemples d'huiles siliconées, on peut notamment citer les polydiméthylsiloxanes (PDMS), les polyorganosiloxanes phénylés tels que les phényltriméthicones, les phényltriméthylsiloxy-diphénylsiloxanes, les diphénylméthyldiméthyltrisiloxanes, les diphényldiméthicones, les phényldiméthicones, les polyméthylphénylsiloxanes, éventuellement fluorés ; les polysiloxanes modifiés par des acides gras, des alcools gras ou des polyoxyalkylènes, les silicones fluorées, les huiles siliconées perfluorées, les huiles de silicones aminées.

Parmi les huiles siliconées préférées, on peut citer les polydiméthysiloxanes, les polyméthylphénylsiloxanes, et leurs mélanges.

Ces huiles siliconées peuvent comporter éventuellement des groupes alkyle, hydroxy ou alcoxy en bout de chaîne siliconée ou pendante, comme par exemple, les polydiméthylsiloxanes à groupements terminaux triméthylsilyle et polydiméthylsiloxanes à groupements terminaux diméthylsilanol.

A titre d'exemples de polydiméthylsiloxanes à groupements terminaux triméthylsilyle, on peut notamment citer ceux ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHODIA ;
- les huiles de la série 200 de la société DOW CORNING telles que plus particulièrement la DC200 de viscosité 60 000 cSt;
- les huiles vendues sous la dénomination commerciale Belsil DM 300000 et Belsil DM 60000 par la société Wacker Chemie AG ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA.

Comme huile siliconée utilisable dans l'invention, on peut encore citer les silicones linéaires ou cycliques, et comportant en particulier de 2 à 7 atomes de silicium. On peut notamment citer l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et leurs mélanges.

Les huiles particulièrement préférées dans le cadre de la présente invention sont les polydiméthylsiloxanes et leurs mélanges.

La ou les huile(s) telle(s) que définie(s) ci-dessus est ou sont de préférence présente(s) en une quantité comprise entre 0,1 et 50 % en poids, encore plus préférentiellement en une quantité comprise entre 1 et 30 % en poids par rapport au poids de la composition.

La composition selon l'invention peut comprendre en outre une ou plusieurs huiles végétales.

En particulier, on entend par "huile végétale" une huile telle que définie ci-dessus, extraite d'une espèce appartenant au règne végétal.

L'huile végétale ou les huiles végétales utilisées selon l'invention sont différentes des alcanes linéaires volatils tels que définis ci-avant et sont choisies parmi les huiles végétales habituellement employées dans le domaine cosmétique.

A titre d'exemples d'huile végétale utilisable dans les compositions de l'invention, on peut citer l'huile de cameline, l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyau d'abricot, l'huile d'olive, l'huile d'onagre, l'huile de palme, l'huile de passiflore, l'huile de pépins de raisin, l'huile de rosier, l'huile de ricin, l'huile de seigle, l'huile de sésame, l'huile de son de riz, l'huile de soja, l'huile de tournesol, et leurs mélanges.

Parmi les huiles végétales citées ci-dessus, on utilise de préférence l'huile de cameline, l'huile d'amande douce, l'huile d'olive, l'huile d'argan, l'huile d'avocat, l'huile de colza, l'huile de jojoba ou cire liquide de jojoba, l'huile de soja, l'huile de tournesol, et de manière plus préférée l'huile de cameline, l'huile d'amande douce, l'huile d'avocat, l'huile de jojoba ou cire liquide de jojoba, et leurs mélanges, et mieux encore l'huile de cameline, l'huile d'amande douce, et leurs mélanges.

La composition selon l'invention peut comprendre en outre un ou plusieurs additifs classiques bien connus dans la technique, tels que des polymères associatifs ou non, ioniques, à savoir cationiques ou anioniques, non-ioniques, ou amphotères, des alcools gras en C₁₀₋₃₀, des polyols, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des agents nacrants, des propulseurs, et des épaississants minéraux ou organiques.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions selon l'invention peuvent se présenter sous la forme d'une lotion plus ou moins épaissie, ou d'un gel.

L'invention est également relative à un procédé de traitement cosmétique des matières kératiniques, de préférence des fibres kératiniques comme les cheveux, qui consiste à appliquer une quantité efficace d'une composition cosmétique telle que décrite ci-dessus, sur lesdites matières, à éventuellement rincer après un éventuel temps de pause.

Lorsque l'on applique la composition selon l'invention, on la laisse éventuellement pauser sur les cheveux pendant environ 1/2 min. à 5 minutes, puis on rince éventuellement à l'eau.

Les exemples suivants sont donnés à titre illustratif de la présente invention.

Dans les exemples suivants, toutes les quantités sont indiquées en pour cent en poids de matière en l'état par rapport au poids total de la composition, sauf indication contraire.

### EXEMPLES

### Exemple 1

On a préparé la composition de soin non rincé suivante à partir des ingrédients indiqués dans le tableau ci-dessous.

| | |
|---|---|
| Myristate d'isopropyle | 77,8 % |
| Polydiméthylsiloxane vendu sous la dénomination commerciale Belsil DM 300000 par la société Wacker Chemie AG | 20 % |
| Mélange majoritairement composé de n-undécane et de n-tridécane selon l'exemple 2 de la demande WO 2008/155059. | 2 % |
| Parfum | 0,2 % |

On a appliqué cette composition sur les cheveux. On a observé d'excellentes performances de lissage et de brillance.

### Exemple 2

On a préparé la composition de soin non rincé suivante à partir des ingrédients indiqués dans le tableau ci-dessous.

| | |
|---|---|
| Myristate d'isopropyle | 75 % |
| Polydiméthylsiloxane vendu sous la dénomination commerciale Belsil DM 300000 par la société Wacker Chemie AG | 5 % |
| Polydiméthylsiloxane vendu sous la dénomination commerciale Wacker Belsil DM 60000 par la société Wacker Chemie AG | 1 % |
| Mélange majoritairement composé de n-undécane et de n-tridécane selon l'exemple 2 de la demande WO 2008/155059 | 18,8 % |
| Parfum | 0,2 % |

On a appliqué cette composition sur les cheveux. On a observé une meilleure facilité de mise en forme.

### Exemple 3

On a préparé la composition de soin non rincé suivante à partir des ingrédients indiqués dans le tableau ci-dessous.

| | |
|---|---|
| Myristate d'isopropyle | 50 % |
| Polydiméthylsiloxane vendu sous la dénomination commerciale Belsil DM 300000 par la société Wacker Chemie AG | 4 % |
| Polydiméthylsiloxane vendu sous la dénomination commerciale Wacker Belsil DM 60000 par la société Wacker Chemie AG | 3 % |
| Mélange de n-undécane et de n-tridécane selon l'exemple 2 de la demande WO 2008/155059 | 42,8 % |
| Parfum | 0,2 % |

On a appliqué cette composition sur les cheveux. On a observé une meilleure facilité de mise en forme, ainsi que d'excellentes performances de lissage visuel et toucher.

### Exemple 4

On a préparé la composition de soin non rincé suivante à partir des ingrédients indiqués dans le tableau ci-dessous.

| | |
|---|---|
| Myristate d'isopropyle | 15 % |
| Huile d'amande douce | 7 % |
| Triglycérides d'acides caprylique/caprique (60/40) (MYRITOL 318 de COGNIS) | 15 % |
| Huile de caméline raffinée | 7 % |
| Mélange majoritairement composé de n-undécane et de n-tridécane selon l'exemple 2 de la demande WO 2008/155059 | 55 % |
| Parfum | 1 % |

On a appliqué cette composition sur les cheveux. On a observé un excellent toucher des cheveux humides et secs, qui se traduit notamment par un transfert moindre de la composition sur les mains, après passage des mains dans les cheveux.

### Exemple 5

On a préparé la composition de soin non rincé suivante à partir des ingrédients indiqués dans le tableau ci-dessous.

| | |
|---|---|
| Myristate d'isopropyle | 14 % |
| Polydiméthylsiloxane vendu sous la dénomination commerciale Belsil DM 300000 par la société Wacker Chemie AG | 15,4 % |
| Mélange majoritairement composé de n-undécane et de n-tridécane selon l'exemple 2 de la demande WO 2008/155059 | 64,9 % |
| 4-méthoxycinnamate de 2-éthylhydexyle (PARSOL MCX de DSM NUTRITIONAL PRODUCT) | 0,5 % |
| Ethanol | 5 % |
| Parfum | 0,2 |

On a appliqué cette composition sur les cheveux. On a observé d'excellentes propriétés de lissage, toucher et légèreté.

### Exemple 6

On a préparé la composition de soin non rincé suivante à partir des ingrédients indiqués dans le tableau ci-dessous.

| | |
|---|---|
| Myristate d'isopropyle | 50 % |
| Polydiméthylsiloxane vendu sous la dénomination commerciale Belsil DM 300000 par la société Wacker Chemie AG | 4 % |
| Polydiméthylsiloxane vendu sous la dénomination commerciale Wacker Belsil DM 60000 par la société Wacker Chemie AG | 3 % |
| Mélange de n-dodécane et de n-tétradécane vendu sous la dénomination commerciale Vegelight 1214 par la société Biosynthis | 42,8 % |
| Parfum | 0,2 % |

On a appliqué cette composition sur les cheveux. On a observé une meilleure facilité de mise en forme, ainsi que d'excellentes performances de lissage visuel et toucher.

## Revendications

1. Utilisation pour le traitement cosmétique des fibres kératiniques comme les cheveux, d'un ou plusieurs alcanes linéaires volatils comportant de 7 à 15 atomes de carbone et d'un ou plusieurs esters gras, liquides à 25°C et à pression atmosphérique, de mono- ou diacide carboxylique en C₁₂₋₃₀ et de monoalcool en C ₁₋₃₀ en milieu anhydre, dans un rapport pondéral alcane(s) linéaire(s) volatil(s)/ester(s) gras liquide(s) inférieur à 5.

2. Utilisation selon la revendication 1, **caractérisée en ce que** l'alcane linéaire volatil est choisi parmi le n- heptane (C7), le n-octane (C8), le n-nonane (C9), le n-décane (C10), le n-undécane (C11), le n-dodécane (C12), le n-tridécane (C13), le n-tétradécane (C14), le n-pentadécane (C15) et leurs mélanges.

3. Utilisation selon la revendication 2, **caractérisée en ce que** l'alcane linéaire volatil est choisi parmi le n-nonane, le n-undécane, le n-dodécane, le n-tridécane, le n-tétradécane, et leurs mélanges.

4. Utilisation selon l'une quelconque des revendications précédentes, **caractérisée en ce que** l'alcane linéaire volatil est d'origine végétale.

5. Utilisation selon l'une quelconque des revendications précédentes, **caractérisée en ce que** l'ester gras liquide de mono- ou diacide carboxylique en C₁₂₋₃₀ et de monoalcool en C₁₋₃₀ est choisi parmi le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate de 2-octyldodécyle, le monococoate de 2-éthylhexyle (ou monococoate d'octyle), le palmitate d'éthyle, le palmitate d'isopropyle, le palmitate d'isobutyle, le palmitate de 2-éthylhexyle (ou palmitate d'octyle), le stéarate de butyle, le stéarate d'isopropyle, le stéarate d'isobutyle, le stéarate d'isocétyle, l'isostéarate d'isotéaryle, l'isostéarate d'isopropyle, le stéarate de 2-éthylhexyle (ou stéarate d'octyle), l'hydroxystéarate de 2-éthylhexyle (ou hydroxystéarate d'octyle), l'oléate de décyle, le sébacate de diisopropyle, l'isononanoate d'isononyle, le néopentanoate de tridécyle, le néopentanoate d'isocétyle, le néopentanoate d'isostéaryle, le néopentanoate d'octyldodécyle et le néopentanoate d'isoarachidyle et leurs mélanges.

6. Utilisation selon l'une quelconque des revendications précédentes, **caractérisée en ce que** le rapport pondéral alcane(s) linéaire(s) volatil(s)/ester(s) gras liquide(s) va de 0,1 à 4,9.

7. Composition cosmétique comprenant, dans un milieu anhydre :
- un ou plusieurs alcanes linéaires volatils comportant de 7 à 15 atomes de carbone,
- un ou plusieurs esters gras liquides à 25°C et à pression atmosphérique, d'un mono- ou diacide carboxylique en C₁₂₋₃₀ et d'un mono-alcool en C ₁₋₃₀ en un rapport pondéral alcane(s) linéaire(s) volatil(s)/ester(s) gras liquide(s) supérieur ou égal à 0,1 et inférieur à 5, et
- une ou plusieurs huiles choisies parmi les huiles siliconées.

8. Composition cosmétique selon la revendication 7, **caractérisée en ce que** le ou les alcanes linéaires volatils sont présents en une teneur allant de 0,5 % à 90 % en poids, en particulier de 1 % à 70 % en poids, et plus particulièrement de 2 % à 70 % en poids, et mieux de 15% à 70% en poids par rapport au poids total de la composition.

9. Composition cosmétique selon la revendication 7 ou 8, **caractérisée en ce que** le ou les esters gras liquides à 25°C et à pression atmosphérique sont choisis parmi le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate de 2-octyldodécyle, le monococoate de 2-éthylhexyle (ou monococoate d'octyle), le palmitate d'éthyle, le palmitate d'isopropyle, le palmitate d'isobutyle, le palmitate de 2-éthylhexyle (ou palmitate d'octyle), le stéarate de butyle, le stéarate d'isopropyle, le stéarate d'isobutyle, le stéarate d'isocétyle, l'isostéarate d'isotéaryle, l'isostéarate d'isopropyle, le stéarate de 2-éthylhexyle (ou stéarate d'octyle), l'hydroxystéarate de 2-éthylhexyle (ou hydroxystéarate d'octyle), l'oléate de décyle, le sébacate de diisopropyle, l'isononanoate d'isononyle, le néopentanoate de tridécyle, le néopentanoate d'isocétyle, le néopentanoate d'isostéaryle, le néopentanoate d'octyldodécyle et le néopentanoate d'isoarachidyle et leurs mélanges.

10. Composition cosmétique selon l'une quelconque des revendications 7 à 9, **caractérisée en ce que** le ou les esters gras liquide(s) d'un mono- ou diacide carboxylique en C₁₂₋₃₀ et d'un mono-alcool en C₁₋₃₀ est ou sont présent(s) en une quantité allant de 0,1 à 90% en poids, de préférence de 1 à 80% en poids, et mieux de 10 à 80% en poids par rapport au poids total de la composition.

11. Composition cosmétique selon l'une quelconque des revendications 7 à 10, **caractérisée en ce que** les huiles siliconées sont choisies parmi les polydiméthylsiloxanes, les polyméthylphénylsiloxanes, et leurs mélanges.

12. Composition cosmétique selon l'une quelconque des revendications 7 à 11, **caractérisée en ce que** la ou les huile(s) est ou sont présente(s) en une quantité comprise entre 0,1 et 50% en poids, de préférence entre 1 et 30% en poids par rapport au poids de la composition.

13. Procédé de traitement des matières kératiniques, de préférences des fibres kératiniques, et mieux encore des cheveux, comprenant l'application de la composition cosmétique selon l'une quelconque des revendications 7 à 12.

## Patentansprüche

1. Verwendung eines oder mehrerer flüchtiger linearer Alkane mit 7 bis 15 Kohlenstoffatomen und eines oder mehrerer bei 25°C und Normaldruck flüssiger Fettester von C₁₂₋₃₀-Mono- oder -Dicarbonsäure und C₁₋₃₀-Monoalkohol in wasserfreiem Medium in einem Gewichtsverhältnis von flüchtigem linearem Alkan bzw. flüchtigen linearen Alkanen zu flüssigem Fettester bzw. flüssigen Fettestern von weniger als 5 zur kosmetischen Behandlung von Keratinfasern wie Haaren.

2. Verwendung nach Anspruch 1, **dadurch gekennzeichnet, dass** das flüchtige lineare Alkan aus n-Heptan (C7), n-Octan (C8), n-Nonan (C9), n-Decan (C10), n-Undecan (C11), n-Dodecan (C12), n-Tridecan (C13), n-Tetradecan (C14), n-Pentadecan (C15) und Mischungen davon ausgewählt ist.

3. Verwendung nach Anspruch 2, **dadurch gekennzeichnet, dass** das flüchtige lineare Alkan aus n-Nonan, n-Undecan, n-Dodecan, n-Tridecan, n-Tetradecan und Mischungen davon ausgewählt ist.

4. Verwendung nach einem der vorhergehenden Ansprüche, **dadurch gekennzeichnet, dass** das flüchtige lineare Alkan pflanzlichen Ursprungs ist.

5. Verwendung nach einem der vorhergehenden Ansprüche, **dadurch gekennzeichnet, dass** der flüssige Fettester von C₁₂₋₃₀-Mono- oder - Dicarbonsäure und C₁₋₃₀-Monoalkohol aus Ethyllaurat, Butyllaurat, Hexyllaurat, Isohexyllaurat, Isopropyllaurat, Methylmyristat, Ethylmyristat, Butylmyristat, Isobutylmyristat, Isopropylmyristat, 2-Octyldodecylmyristat, 2-Ethylhexylmonococoat (oder Octylmonococoat), Ethylpalmitat, Isopropylpalmitat, Isobutylpalmitat, 2-Ethylhexylpalmitat (oder Octylpalmitat), Butylstearat, Isopropylstearat, Isobutylstearat, Isocetylstearat, Isostearylisostearat, Isopropylisostearat, 2-Ethylhexylstearat (oder Octylstearat), 2-Ethylhexylhydroxystearat (oder Octylhydroxystearat), Decyloleat, Diisopropylsebacat, Isononylisononanoat, Tridecylneopentanoat, Isocetylneopentanoat, Isostearylneopentanoat, Octyldodecylneopentanoat und Isoarachidylneopentanoat und Mischungen davon ausgewählt ist.

6. Verwendung nach einem der vorhergehenden Ansprüche, **dadurch gekennzeichnet, dass** das Gewichtsverhältnis von flüchtigem linearem Alkan bzw. flüchtigen linearen Alkanen zu flüssigem Fettester bzw. flüssigen Fettestern größer gleich 0,1 und kleiner gleich 4,9 ist.

7. Kosmetische Zusammensetzung, umfassend in einem wasserfreien Medium:
- ein oder mehrere flüchtige lineare Alkane mit 7 bis 15 Kohlenstoffatomen,
- einen oder mehrere bei 25°C und Normaldruck flüssige Fettester von C₁₂₋₃₀-Mono- oder -Dicarbonsäure und C₁₋₃₀-Monoalkohol in einem Gewichtsverhältnis von flüchtigem linearem Alkan bzw. flüchtigen linearen Alkanen zu flüssigem Fettester bzw. flüssigen Fettestern von größer gleich 0,1 und kleiner als 5 und
- ein oder mehrere Öle, die aus Silikonölen ausgewählt sind.

8. Kosmetische Zusammensetzung nach Anspruch 7, **dadurch gekennzeichnet, dass** das bzw. die flüchtigen linearen Alkane in einem Gehalt im Bereich von 0,5 bis 90 Gew.-%, insbesondere 1 bis 70 Gew.-% und spezieller 2 bis 70 Gew.-% und besser 15 bis 70 Gew.-%, bezogen auf das Gesamtgewicht der Zusammensetzung, vorliegen.

9. Kosmetische Zusammensetzung nach Anspruch 7 oder 8, **dadurch gekennzeichnet, dass** der bzw. die bei 25°C und Normaldruck flüssigen Fettester aus Ethyllaurat, Butyllaurat, Hexyllaurat, Isohexyllaurat, Isopropyllaurat, Methylmyristat, Ethylmyristat, Butylmyristat, Isobutylmyristat, Isopropylmyristat, 2-Octyldodecylmyristat, 2-Ethylhexylmonococoat (oder Octylmonococoat), Ethylpalmitat, Isopropylpalmitat, Isobutylpalmitat, 2-Ethylhexylpalmitat (oder Octylpalmitat), Butylstearat, Isopropylstearat, Isobutylstearat, Isocetylstearat, Isostearylisostearat, Isopropylisostearat, 2-Ethylhexylstearat (oder Octylstearat), 2-Ethylhexylhydroxystearat (oder Octylhydroxystearat), Decyloleat, Diisopropylsebacat, Isononylisononanoat, Tridecylneopentanoat, Isocetylneopentanoat, Isostearylneopentanoat, Octyldodecylneopentanoat und Isoarachidylneopentanoat und Mischungen davon ausgewählt sind.

10. Zusammensetzung nach einem der Ansprüche 7 bis 9, **dadurch gekennzeichnet, dass** der bzw. die flüssigen Fettester von C₁₂₋₃₀-Mono- oder - Dicarbonsäure und C₁₋₃₀-Monoalkohol in einer Menge von 0,1 bis 90 Gew.-%, vorzugsweise 1 bis 80 Gew.-% und besser 10 bis 80 Gew.-%, bezogen auf das Gesamtgewicht der Zusammensetzung, vorliegt bzw. vorliegen.

11. Kosmetische Zusammensetzung nach einem der Ansprüche 7 bis 10, **dadurch gekennzeichnet, dass** die Silikonöle aus Polydimethylsiloxanen, Polymethylphenylsiloxanen und Mischungen davon ausgewählt sind.

12. Kosmetische Zusammensetzung nach einem der Ansprüche 7 bis 11, **dadurch gekennzeichnet, dass** das bzw. die Öle in einer Menge zwischen 0,1 und 50 Gew.-%, vorzugsweise zwischen 1 und 30 Gew.-%, bezogen auf das Gesamtgewicht der Zusammensetzung, vorliegt bzw. vorliegen.

13. Verfahren zur Behandlung von Keratinmaterialien, vorzugsweise Keratinfasern und noch besser Haaren, bei dem man die kosmetische Zusammensetzung nach einem der Ansprüche 7 bis 12 aufbringt.

## Claims

1. Use, for the cosmetic treatment of keratin fibres such as the hair, of one or more volatile linear alkanes having from 7 to 15 carbon atoms and of one or more fatty esters, which are liquid at 25 °C and atmospheric pressure, of C₁₂₋₃₀ mono- or dicarboxylic acid and of C₁₋₃₀ monohydric alcohol in an anhydrous medium, in a weight ratio of volatile linear alkane (s) to liquid fatty ester(s) of less than 5.

2. Use according to Claim 1, **characterized in that** the volatile linear alkane is selected from n-heptane (C7), n-octane (C8), n-nonane (C9), n-decane (C10), n-undecane (C11), n-dodecane (C12), n-tridecane (C13), n-tetradecane (C14), n-pentadecane (C15) and mixtures thereof.

3. Use according to Claim 2, **characterized in that** the volatile linear alkane is selected from n-nonane, n-undecane, n-dodecane, n-tridecane, n-tetradecane, and mixtures thereof.

4. Use according to any one of the preceding claims, **characterized in that** the volatile linear alkane is of vegetable origin.

5. Use according to any one of the preceding claims, **characterized in that** the liquid fatty ester of C₁₂₋₃₀ mono- or dicarboxylic acid and of C₁₋₃₀ monohydric alcohol is selected from ethyl laurate, butyl laurate, hexyl laurate, isohexyl laurate, isopropyl laurate, methyl myristate, ethyl myristate, butyl myristate, isobutyl myristate, isopropyl myristate, 2-octyldodecyl myristate, 2-ethylhexyl monococoate (or octyl monococoate), ethyl palmitate, isopropyl palmitate, isobutyl palmitate, 2-ethylhexyl palmitate (or octyl palmitate), butyl stearate, isopropyl stearate, isobutyl stearate, isocetyl stearate, isostearyl isostearate, isopropyl isostearate, 2-ethylhexyl stearate (or octyl stearate), 2-ethylhexyl hydroxystearate (or octyl hydroxystearate), decyl oleate, diisopropyl sebacate, isononyl isononanoate, tridecyl neopentanoate, isocetyl neopentanoate, isostearyl neopentanoate, octyldodecyl neopentanoate and isoarachidyl neopentanoate and mixtures thereof.

6. Use according to any one of the preceding claims, **characterized in that** the weight ratio of volatile linear alkane (s) to liquid fatty ester(s) is in the range from 0.01 to 4.9.

7. Cosmetic composition comprising, in an anhydrous medium:
- one or more volatile linear alkanes having from 7 to 15 carbon atoms,
- one or more fatty esters, which are liquid at 25°C and atmospheric pressure, of a C₁₂-₃₀ mono- or dicarboxylic acid and of a C₁₋₃₀ monohydric alcohol in a weight ratio of volatile linear alkane (s) to liquid fatty ester (s) of greater than or equal to 0.1 and less than 5, and
- one or more oils selected from silicone oils.

8. Cosmetic composition according to Claim 7, **characterized in that** the volatile linear alkane or alkanes are present in a content in the range from 0.5 to 90 wt.%, in particular from 1 to 70 wt.%, and more particularly from 2 to 70 wt.%, and preferably from 15 to 70 wt.% relative to the total weight of the composition.

9. Cosmetic composition according to Claim 7 or 8, **characterized in that** the fatty ester or esters, which are liquid at 25°C and atmospheric pressure, are selected from ethyl laurate, butyl laurate, hexyl laurate, isohexyl laurate, isopropyl laurate, methyl myristate, ethyl myristate, butyl myristate, isobutyl myristate, isopropyl myristate, 2-octyldodecyl myristate, 2-ethylhexyl monococoate (or octyl monococoate), ethyl palmitate, isopropyl palmitate, isobutyl palmitate, 2-ethylhexyl palmitate (or octyl palmitate), butyl stearate, isopropyl stearate, isobutyl stearate, isocetyl stearate, isostearyl isostearate, isopropyl isostearate, 2-ethylhexyl stearate (or octyl stearate), 2-ethylhexyl hydroxystearate (or octyl hydroxystearate), decyl oleate, diisopropyl sebacate, isononyl isononanoate, tridecyl neopentanoate, isocetyl neopentanoate, isostearyl neopentanoate, octyldodecyl neopentanoate and isoarachidyl neopentanoate and mixtures thereof.

10. Cosmetic composition according to any one of Claims 7 to 9, **characterized in that** the liquid fatty ester or esters of a C₁₋₃₀ mono- or dicarboxylic acid and of a C₁₂₋₃₀ monohydric alcohol is/are present in an amount in the range from 0.1 to 90 wt.%, preferably from 1 to 80 wt.%, and more preferably from 10 to 80 wt.% relative to the total weight of the composition.

11. Cosmetic composition according to any one of Claims 7 to 10, **characterized in that** the silicone oils are selected from polydimethylsiloxanes, polymethylphenylsiloxanes, and mixtures thereof.

12. Cosmetic composition according to any one of Claims 7 to 11, **characterized in that** the oil(s) is/are present in an amount between 0.1 and 50 wt.%, preferably between 1 and 30 wt.% relative to the weight of the composition.

13. Method of treatment of keratinous materials, preferably of keratin fibres, and more preferably of the hair, comprising application of the cosmetic composition according to any one of Claims 7 to 12.
